# EUROPEAN PATENT APPLICATION

(11) **EP 1 172 119 A2**
(43) Date of publication of application: **16.01.2002**
(21) Application number: 01116297.1
(22) Date of filing: 05.07.2001
(51) Int. Cl.: A61L 9/12

(54) **Air deodorising and/or perfuming group**

(30) Priority: 13.07.2000 IT VI000143
(71) Applicant: Indest s.r.l., 36010 Velo d'Astico (Vicenza) (IT)
(72) Inventor: Dal Pra', Robert, 36010 Cogollo Del Cengio (Vicenza) (IT)
(74) Representative: Bettello, Pietro, Dott. Ing.

(57) **Abstract**

Regards an air deodorising and/or perfuming group, adapted to be used in civil and industrial environments where regular and controlled treatment of the air is required. Such a group is characterised in that it comprises an electromechanical apparatus which conveys a minimum flow rate of air through a duct into which a cartridge filled with scented oils or essences is inserted.

## Description

The finding regards an air deodorising and/or perfuming group, that couples with an air purifier or with other similar apparatus, as described in claim 1.

As is well known, in both civil and industrial environments so called air purifiers are installed which, through an electrostatic or mechanical filtration system, seek to extract harmful substances, such as dust or suspended fumes, from the air.

In terms of construction an air purifier is made up of a case within which is contained, besides a specific filtering system, a fan which seeks to activate the air flow and an electronic control unit inside the apparatus.

In given situations, as well as being purified, the air needs to be deodorised to neutralise possible bad odours, and, often perfumed, to make the environment more pleasant.

At the present state of the art, to deodorise and/or perfume the air, cartridges filled with scented oils or essences which are placed in various parts of the environment to be treated are on the market and are widely used.

Such a system presents the inconvenience that it cannot guarantee a regular and uniform deodorisation and/or perfuming in the environment since the air flow is never regular, being created by the normal convectional circulation of the air in the environment, in which there may or may not be air circulation.

The purpose of the present finding is to realise a mechanism which eliminates such a drawback, which is able to regulate a deodorised and/or perfumed air flow, so that it can spread uniformly inside the environment.

A further purpose of the finding is to realise a mechanism which is easily constructed and of a minimal encumbrance so that it can be coupled with and/or inserted into apparatus for forced air circulation, such as purifiers, conditioners, thermoconvectors and other similar products, also produced normally.

All this is achieved with a group air purifier/perfumer, made up of an electromechanic apparatus which conveys the air at a minimum flow rate through a duct into which a cartridge filled with scented oils or essence is inserted.

The same said group is equipped with an electronic system to control the velocity and the flow rate of the air emitted which goes through the cartridge, which takes place activating the electric fan of the main apparatus, with which said group is coupled.

The same said group can also regulate the quantity of deodorising and/or perfumed air to be released into the environment, which takes place by regulating the position of a shutter inserted in the inlet duct of the deodorising and/or perfumed air flow.

Finally, it is foreseen that the deodorising/perfumed air flow which comes out from the deodorising/perfuming group mixes with the purified air which comes out from the main purifying apparatus, or elso it releases into the environment independently.

The air flow which goes through the deodorising/perfuming group can be taken in directly from outside, or else taken from the purified air flow which comes out of the main apparatus.

In such a first case, the air not having been purified but only prefiltered through a metallic filter, the deodorising/perfuming group needs to be equipped with a further filter, positioned in front of the deodorising cartridge to avoid it being contaminated by the polluting agents present in the air taken in.

These and other characteristics of the finding are largely backed up by the description of one of its possible embodiments, given only as an illustrative and not limiting example, with the help of attached drawings, where:
- fig.1 (Table I) represents a general view of the group according to the finding coupled with a main apparatus, which is an air purifier;
- fig.2 (Table II) shows an overall and partially sectioned perspective view of the group according to the finding;
- fig.3 (Table III) shows an exploded view of the group according to fig.2.
- fig.4 (Table IV) shows a block diagram of the operation of the group according to the finding coupled with an air purifier.

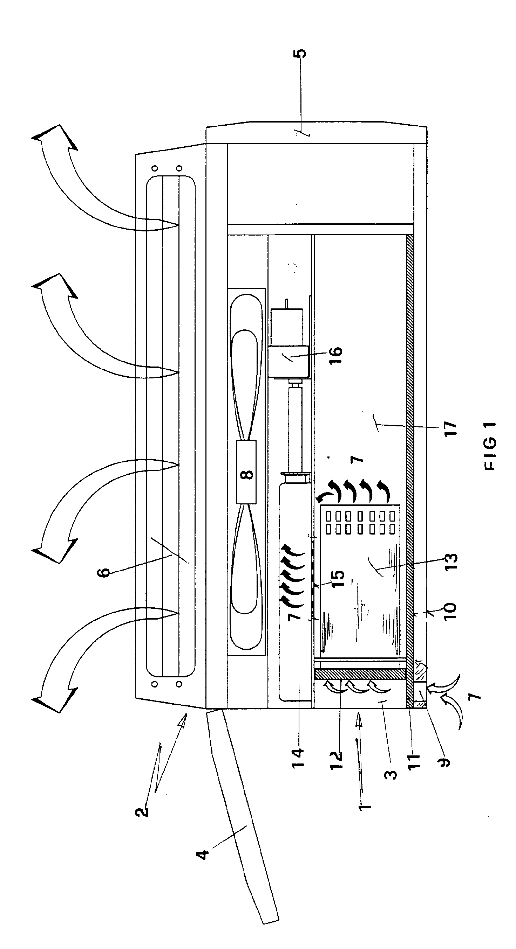

As can be seen in fig.1, the deodorising/perfuming group 1 according to the finding is coupled with an air purifier 2, of normal production.

Specifically, said group 1 is inserted in a space 3, closed by the door 4, located on the casing 5 of said purifier 2, so that the purified air flow and the deodorising and/or perfumed air flow are released into the external atmosphere through a single aperture 6, provided with controllable fins, of the such that all purifiers on the market are eqipped with.

The circulation of the air flow 7 (arrows in fig.1) takes place through an electric fan 8, preferibly of the helicoid type, which can be specifically designed for the operation of the aforementionned group; alternatively the one fixed to the main purifier is used.

Still with reference to fig.1, the air flow 7, drawn in from the outside through the openings 9, located on the protection grill 10, is firstly filtered by the metallic filter 11 and then by the active carbon filter 12, positioned immediately in front of the cartridge 13.

The amount of air 7 which goes through the cartridge 13 is controlled through a shutter 14 which, from partially to totally, shuts off opening 15 which allows the emission of the air flow.

The open and shut movement of the shutter 14 is realised through a driving means 16, connected to the bearing structure of the group.

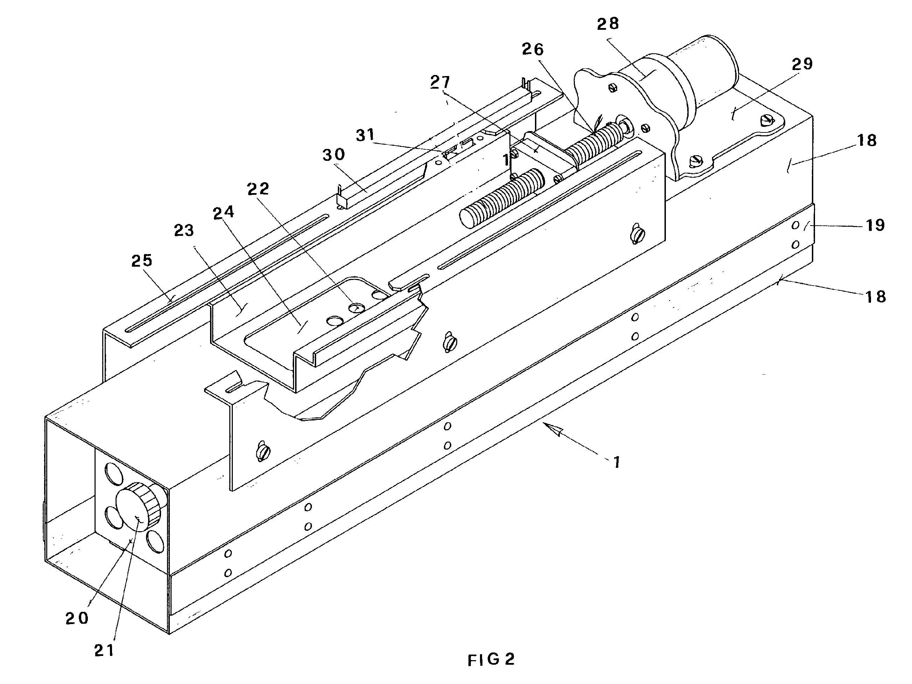

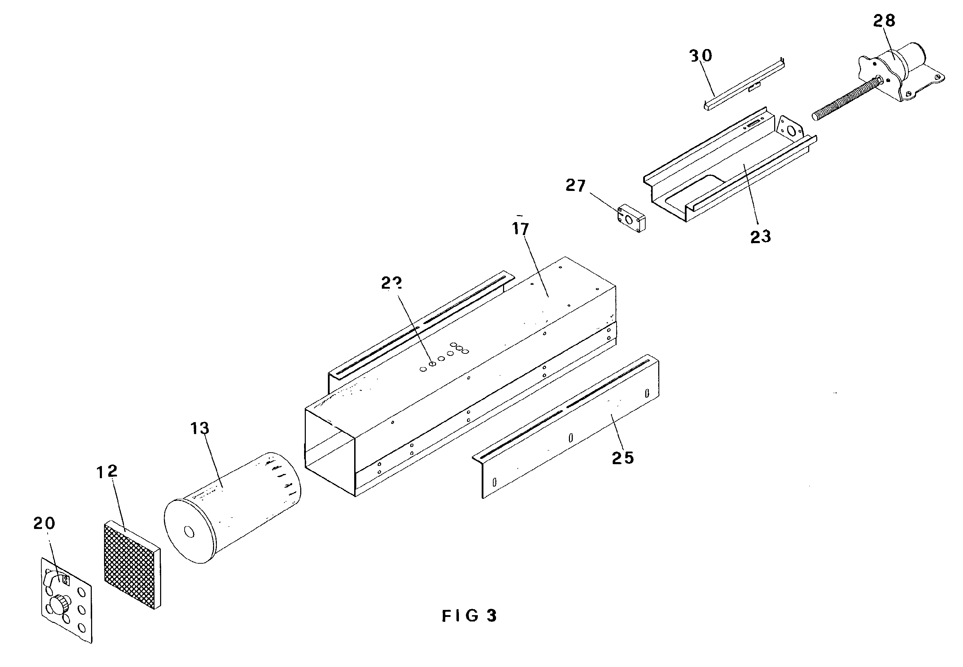

One specific possible embodiment of the deodorising and/or perfuming group according to the invention is shown in figs.2 and 3, where the cartridge 13 is contained inside a duct 17 defined by two half-shells 18 held together by two lateral belts 19.

An active carbon filter 12 is positioned in front of the cartridge 13 and the cartridge space is closed at the front by the flange 20, which is perforated to allow the entry of the air flow 7 and equipped with the knob 21.

On the upper wall of the duct 17 an outlet is provided for the deodorising and/or perfuming air flow, comprised of a plurality of through-holes 22, which are from partially to totally closed off through a shutter comprised of a guillotine 23 equipped, in correspondence with said through-holes, with a losange slit 24.

The alternating rectilinear movement of the guillotine 23, kept in line by the lateral sliding blocks 25, is realised through a threaded pin 26, which screws into the fixed volute 27 and is turned by the motor reducer 28, applied with its base 29 on the wall of the duct 17.

The group is completed with a pair of linear resistors 30 for the positioning of the guillotine and connected to it through the adjustment pins 31.

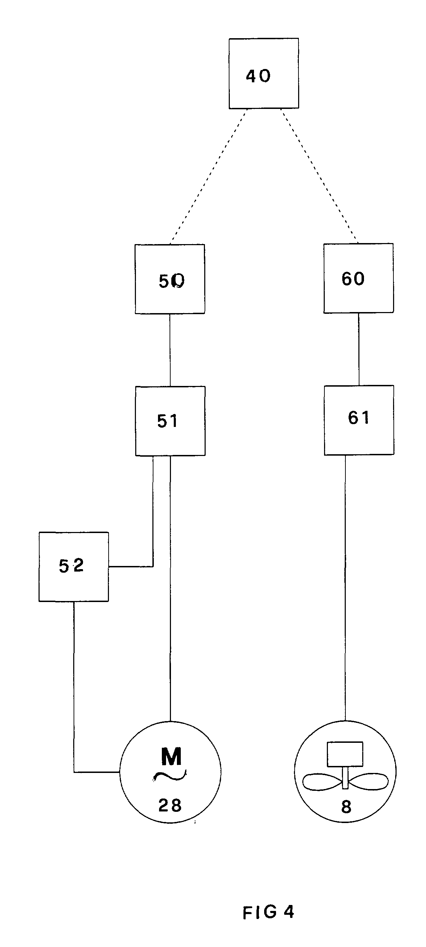

The operation of the whole group described above is shown in the block diagram of fig.4, where the user, using a single infrared, two-channel remote control 40, can control the operation of the deodorising and/or perfuming group and air purifier separately and independently.

In detail, the remote control 40 acts on the displays 50 and 60 which, through their respective control circuits 51 and 61, control the operation of the geared motor 28, which controls the position of the shutter 14, registered through a sensor 52, applied to the linear resistor 20 and the velocity of the electric fan 8 of the purifier.

## Claims

1. AIR DEODORISING AND/OR PERFUMING GROUP, adapted to be used in civil and industrial environments where regular and controlled air treatment is required,
**characterised in that**
it comprises an electromechanical apparatus which conveys a minimum flow rate of air through a duct into which a cartridge filled with scented oils or essences is inserted.

2. GROUP, according to claim 1, **characterised in that** it is coupled with an air purifier, a conditioner or other apparatus used for the treatment of air in civil and industrial environments.

3. GROUP, according to claim 1 and/or 2, **characterised in that** it comprises an electronic control system for the velocity and the flow rate of the air taken in which goes through the cartridge.

4. GROUP, according to claim 1 and/or 2 and 3, **characterised in that** the air flow is controlled by an electric fan applied to the same group.

5. GROUP, according to claim 1 and/or 2 and 3, **characterised in that** the air flow is controlled by the electric fan of the main apparatus, with which said group is coupled.

6. GROUP, according to one or more of the preceding claims, **characterised in that** the air flow emitted by the deodorising/perfuming group mixes with the air flow emitted by the main apparatus, that being an air purifier or other similar apparatus.

7. GROUP, according to one or more of the preceding claims, **characterised in that** the air flow which goes through the cartridge is taken in directly from the outside.

8. GROUP, according to one or more of the preceding claims, **characterised in that** the air flow which goes through the cartridge is taken from the air flow which has been treated, purified or otherwise processed, which is emitted by the main apparatus.

9. GROUP, according to one or more of the preceding claims, **characterised in that** the air flow which goes through the cartridge is adjusted by the position of a shutter inserted into the inlet duct for the deodorising and/or perfumed air flow.

10. AIR DEODORISING AND/OR PERFUMING GROUP, according to one or more of the preceding claims, **characterised in that** it is coupled with an air purifier (2), through its insertion into a space (3), closed by the door (4), formed on the casing (5) of said purifier, so that the purified air flow and the deodorising and/or perfumed air flow are emitted into the external environment through a single aperture (6).

11. GROUP, according to claim 10, **characterised in that** the air flow (7), induced by an electric fan (8), is taken in from outside through the apertures (9) formed on the protection grill (10), filtered firstly by the metallic filter (11) and then by the active carbon filter (12), positioned immediately in front of the cartridge (13).

12. GROUP, according to claims 10 and 11, **characterised in that** the amount of air which goes through the cartridge (13) is adjusted by a shutter (14) which shuts off, from partially to totally, the aperture (15) which allows the emission of the air flow 7, with the open and shut movement of said shutter realised through a driving means (16), fixed to the bearing structure of the group.

13. GROUP, according to claims 10-12, **characterised in that** the cartridge (13) is contained inside a duct (17) defined by two half-shells (18) held together by two lateral belts (19), the cartridge space being closed at the front by the flange (20), perforated to allow the entry of the air flow (7) and equipped with the knob (21).

14. GROUP, according to claims 10-13, **characterised in that** on the upper wall of the duct (17) there is a plurality of through-holes (22) which are from partially to totally shut off by the shutter comprised of a guillotine (23) equipped, in correspondence with said through-holes, with a losenge slit (24).

15. GROUP, according to claim 14, **characterised in that** the alternating rectilinear movement of the guillotine (23), kept in line by the lateral sliding blocks (25), is realised through a threaded pin (26) which screws in to the fixed volute (27) and is turned by the geared motor (28), connected with the base (29) on the wall of the duct (17).

16. GROUP, according to claim 14, **characterised in that** it foresees a pair of linear resistors (30) for positioning the guillotine and connected to it by the adjustment pins (31).

17. OPERATION OF AIR DEODORISING AND/OR PERFUMING GROUP, described in one or more of the preceding claims, **characterised in that** it foresees a single infrared, two-channel remote control (40), to control the operation of the deodorising/perfuming group and the air purifier separately and independently.

18. OPERATION, according to claim 17, **characterised in that** the remote control (40) acts on the displays (50-60) which, through the respective control circuits (51-61), control the operation, respectively, of the geared motor (28), which adjusts the position of the shutter (14), registered by a sensor (52), applied to the linear resistor (20) and the velocity of the electric fan (8) of the purifier.
